# EUROPEAN PATENT SPECIFICATION

(11) **EP 1 154 763 B1**
(45) Date of publication and mention of the grant of the patent: **24.11.2004**
(21) Application number: 00985637.8
(22) Date of filing: 20.12.2000
(51) Int. Cl.: A61K 9/70, A61K 33/34, A61K 7/48, A61N 2/06

(54) **PATCH FOR TRANSDERMAL THERAPY**
PFLASTER ZUR TRANSDERMALTHERAPIE
TIMBRE POUR THERAPIE TRANSDERMIQUE

(30) Priority: 24.12.1999 GB 9930875
(43) Date of publication of application: 21.11.2001
(73) Proprietor: Jehan, David, Birmingham B15 1PT (GB)
(72) Inventor: Jehan, David, Birmingham B15 1PT (GB)
(74) Representative: Carpenter, David
(86) International application number: PCT/GB2000/004896
(87) International publication number: WO 2001/047502

(56) References cited:
- WO-A-92/10154
- GB-A- 2 307 862

## Description

The present invention relates to patch structures for transdermal therapy.

Transdermal patches for supplying a medicament directly through, or onto, the skin of a user are well known. Examples of such patches are patches for hormone replacement therapy and nicotine patches. However, other transdermal patches provide a therapeutic effect by their application to the skin of a user without the presence of a recognised medicament or drug. Examples of such patches are those shown in British Patent 1596314, and my own published PCT Application W097/20549. It is an object of the present invention to provide enhancements of the patch of PCT Application W097/20549.

In accordance with the first aspect of the present invention there is provided a patch for transdermal therapy, the patch comprising at least one layer of sheet material for adhesive attachment to the skin of a user, particulate material including particulate copper or an alloy thereof provided on the surface of said layer of sheet material presented to the skin of the user in use, and, at least one magnet disposed on the opposite surface of said layer of sheet material.

Preferably said magnet is sandwiched between said layer of sheet material and a second layer of sheet material adhesively secured to said opposite surface of the first mentioned layer.

Desirably, said particulate material also includes particulate zinc. Alternatively said patch includes a region of particulate zinc discrete from said particulate copper.

Preferably said discrete regions of copper and zinc communicate so as to be contactable by a common body of liquid electrolyte.

Preferably said particulate zinc is disposed in a generally circular region on said surface of said sheet and is encircled by a generally circular annular region of particulate copper or an alloy thereof.

Preferably a reinforcing strip of sheet material overlies said magnet in association with said second layer.

Preferably, said magnet is annular and particulate zinc material is disposed within the central aperture of the annular magnet.

Desirably, said first layer of sheet material is perforated in alignment with the central aperture of the magnet to provide communication through said first layer of sheet material between the particulate zinc in the central aperture of the magnet and the particulate copper containing material on the face of said layer presented, in use, to the skin of the user.

Preferably said patch includes a peelable carrier sheet upon which the patch is mounted by way of the adhesive surface of said layer of sheet material, and a separate tab of carrier sheet material peelably adhesively secured to a peripheral region of said first layer of sheet material whereby said tab can remain in place on said layer of sheet material when the patch is peeled from the carrier sheet to facilitate handling of the patch prior said tab being peeled from said layer for example when the layer is applied to the skin of the user.

One example of the present invention is illustrated in the accompanying drawings wherein:-
Figure 1 is a diagrammatic cross-sectional view of a transdermal patch,
Figure 2 is a view similar to Figure 1 of a modification,
Figure 3 is an enlarged view in the direction of arrow A in Figure 2,
Figure 4 is an enlarged view similar to Figure 1 of a further modification,
Figures 5, 6 and 7 are views similar to Figure 4 of first, second and third adaptations of the patch of Figure 4,
Figure 8 is a view in the direction of arrow A in Figure 4 with one of the layers of sheet material thereof removed for clarity,
Figure 9 is a view similar to Figure 7 of a fourth adaptation of the patch of Figure 4,
Figure 10 is a view in the direction of arrow B in Figure 9, and
Figure 11 is an inverted plan view of the patch of Figure 4 showing the presence of a tab of backing sheet material.

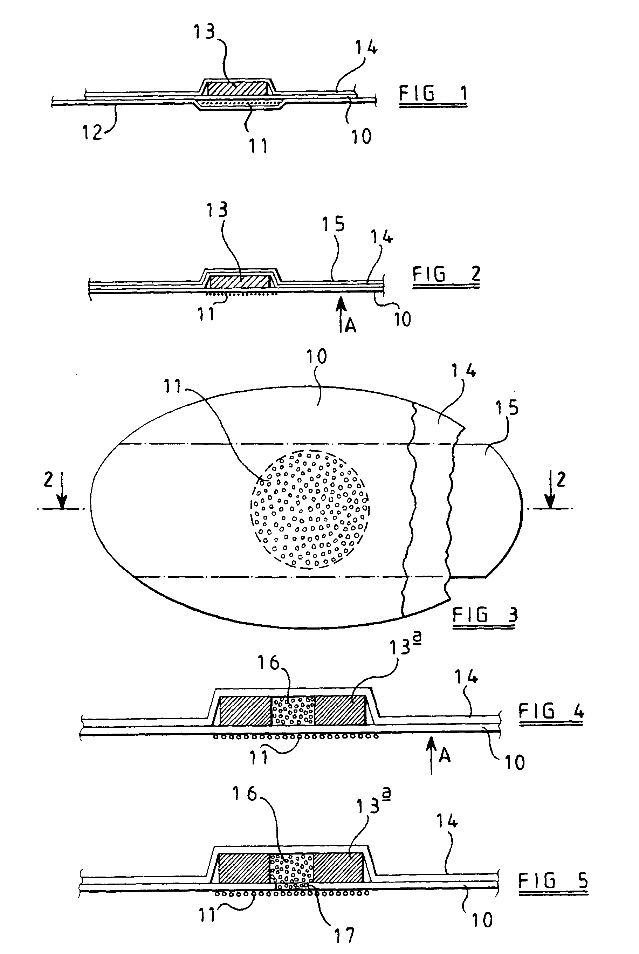

Referring first to Figure 1 of the drawings, the patch comprises a first layer 10 of sheet material which may be any convenient material used in, or suitable for skin contact, for example hypo-allergic material, the layer 10 having on one face thereof a coating of an adhesive material also suitable for skin contact. The material of the layer 10 and the adhesive material may be the same materials used for self-adhesive plasters and wound dressings.

Conveniently, the layer 10 is cut to an elliptical form. Disposed in the geometric centre of the adhesive coated surface of the layer 10 is a generally circular region of particulate material 11. In a basic embodiment the particulate material is copper, or copper alloy, secured to the layer 10 by the adhesive thereon. The particles of the particulate material 11 may be any convenient shape, regular or irregular, symmetrical or non-symmetrical, but conveniently are spherical, and may have the size of between 45 and 1,000 microns. The particulate material 11 may be solid, hollow or porous. In use the adhesive surface of the layer 10 is protected by the layer 10 being mounted on a peelable backing sheet of known form. The backing sheet is illustrated at 12 in Figure 1, but is omitted from the other drawings.

Disposed on the face of the layer 10 opposite the face carrying the particulate material 11, and aligned with the material 11, is a disc like permanent magnet 13. Any form of permanent magnet may be used, but it is preferred that the permanent magnet is in the form of a circular disc of rare-earth material such as samarium or neodymium-iron-boron. The magnet 13 can be secured to the layer 10 by providing an adhesive coating on the face of the layer 10 in the region where the magnet 13 is to be affixed, but more conveniently the magnet 13 is secured in position by a second layer 14 of sheet material similar to the layer 10 and overlying the magnet 13. The adhesive material of the layer 14 secures the layer 14 to the layer 10 and to the magnet 13.

In use, the patch consisting of the layer 10, the particulate material 11, the magnet 13 and the layer 14, is peeled from the backing sheet 12 and is applied to the skin of the user, the particulate material 11 and the adhesive 'lower' face of the layer 10 being presented to the skin of the user so that the adhesive material secures the patch to the skin of the user.

It will be recognised that because the magnet 13 is on the 'outer' face of the layer 10 it does not constitute a spacer spacing the layer 10 from the skin.

The thickness of the particulate material 11 is quite small, and thus there is no 'tenting' effect on the layer 10 which can occur with patches of the kind illustrated in WO97/20549 where the magnet is on the face of the adhesive layer presented to the skin. The effect of 'tenting' tends to facilitate an unintentional detachment of the patch from the skin of the user.

In a modification the particulate material 11 is a mixture of particles of copper or copper alloy, and particles of zinc or zinc alloy, both being in contact with the same body of electrolyte constituted by perspiration from the user.

If desired the layer 14 can be reinforced by a reinforcing strip 15 of material similar to that of the layers 10 and 14. The reinforcing strip is wide enough to overlie the magnet 11 and conveniently extends through the full length of the patch as shown in Figures 2 and 3. Figure 2 illustrates that the reinforcing strip 15 is applied to the outer surface of the layer 14. It is to be understood however that the strip 15 can be interposed between the layer 14 and the layer 10, overlying the magnet 13. The strip 15 is adhesively secured to the layer 14, or to the layer 10 and the magnet 13, dependant upon its location.

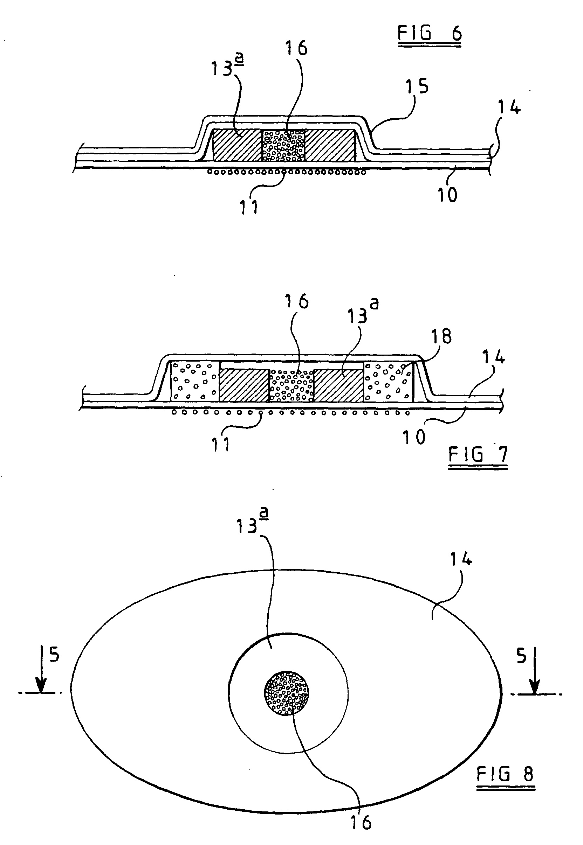

As shown in Figure 4, any of the embodiments described above with reference to Figures 1, 2 and 3 may be provided with a magnet 13a in the form of an annulus of permanent magnet material. The central aperture of the magnet 13a contains particulate zinc 16, the particulate material 11 on the opposite face of the layer 10 being particulate copper or copper alloy. It will be recognised that in the embodiment illustrated in Figure 4 the particulate zinc is always separated from contact with the skin of the wearer by the layer 10. However, the layer 10 is normally porous to perspiration so that perspiration from the users skin can wet the particulate copper 11 and can percolate through the layer 10 to also wet the zinc particles 16. Figure 5 illustrates a modification of the arrangement shown in Figure 4 where the layer 10 is perforated in the region of the central aperture of the magnet 13a. The perforation of the layer 10 can be by way of a single aperture 17 or a plurality of smaller apertures, the objective being to enhance communication between the zinc particulate material 16 and the skin of the wearer through the distribution of copper particles 11. Thus in both embodiments perspiration from the user can percolate through the particulate material 11, through the perforation of the layer 10 and into the particulate material 16 so that both the particulate zinc and the particulate copper/copper alloy are contacted by the same common body of liquid electrolyte defined by such perspiration during use of the patch.

Figure 6 illustrates a modification of the arrangement shown in Figure 4, in that there is a reinforcing strip 15 assisting the layer 14 in overlying the magnet 13a. As described above, the reinforcing strip 15 could be positioned between the layer 14 and the layer 10, overlying the magnet 13a, if desired. Similarly a reinforcing strip 15 could be incorporated in the patch structure of Figure 5.

Figure 7 illustrates the addition of an annulus of resilient foamed synthetic resin material 18 adhesively secured by an adhesive film on the annulus to the outer surface of the layer 10 and surrounding the magnet 13 or 13a. The layer 14 overlies the annulus 18 and the magnet and may, as shown, be adhesively secured to the layer 10, or alternatively the layer 14 could simply be a disc of the same diameter as the annulus 18 secured adhesively to the annulus 18 closing the apertures of both the annulus 18 and the annular magnet. The foam material 18 simply acts as a protective cushion during use of the patch, and it will be appreciated that the annulus 18 can be applied to any of the patch structures described above.

Figure 8 is an inverted plan view of the patch of Figure 4 with the layer 10 omitted.

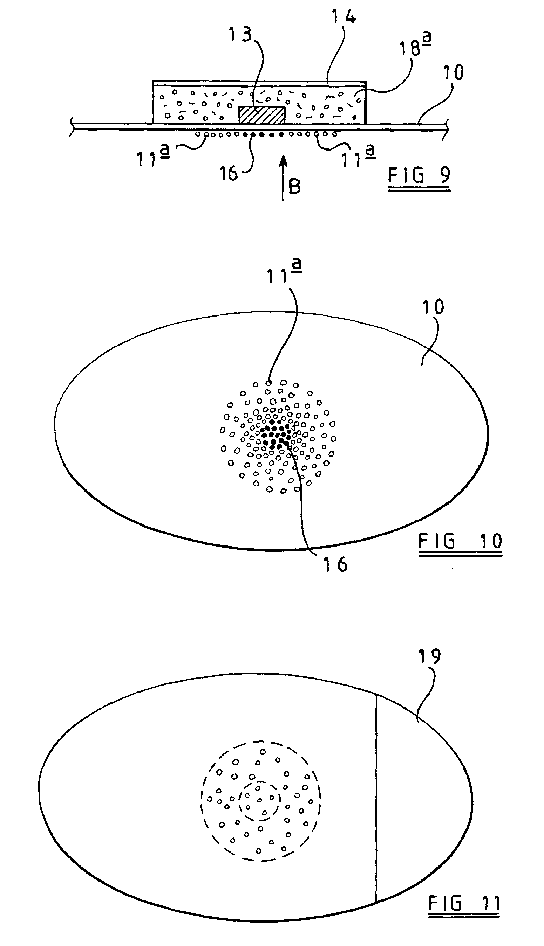

In the patch structure illustrated in Figures 9 and 10 the adhesive carrier layer 10 has, on its adhesive face, a centrally disposed generally circular region 16 of particulate zinc encircled by a generally circular, annular region 11a of particulate copper or copper alloy. Although the regions touch at the outer periphery of the region 16 and the inner periphery of the region 11a, the particulate materials are not significantly intermingled.

Centrally disposed on the opposite face of the layer 10 is a permanent magnet 13 of the kind described above, the permanent magnet 13 being held in place aligned with the region 16 of particulate zinc, by a disc 18a of resilient foamed synthetic resin material. The disc of material 18a is secured to the layer 10 and the magnet 13 by an adhesive coating on the appropriate face of the disc 18a the material of the disc 18a being compressed in the region of the magnet 13 and serving to secure the magnet 13 in position on the layer 10. A second layer 14 of sheet material similar to the layer 10 is disposed on the outer surface of the disc 18a, the layer 14 being cut to a disc of the same diameter. As described above the layer 14 has an adhesive coating on its face presented to the disc 18a to secure the layer 14 to the disc 18a. It will be recognised that if desired the layer 14 could be extended to engage the layer 10 as described above with regard to Figures 4, 5, 6 and 7.

It will be understood that in the arrangement illustrated in Figure 9 and 10 the magnet 13 is completely covered by the combination of the layer 10 and the disc 18a of foamed synthetic resin material, while the particulate material 16, 11a is provided on the "skin" face of the layer 10. The two regions 11a, 16 of particulate material are both in contact with the skin of the user, and thus are both exposed to the same, common body of electrolyte defined by the perspiration of the user.

All of the patch structures described above will preferably be supplied to the user supported on a peelable backing sheet of conventional form. The backing sheet is indicated at 12 in Figure 1, and it will be understood that the same type of backing sheet can be utilised with each of the patch structures described above. The backing sheet will conveniently be a paper or card sheet with a silicone surface to provide 'peelability'. A number of patches may be provided on a single backing sheet, or alternatively each patch may have its own peelable backing sheet.

In order to facilitate removal of a patch from a backing sheet, and subsequent handling of the patch to apply it to the skin of a user, each patch is conveniently provided with a respective tab 19 (Figure 11) of backing sheet material separate from the remainder of the backing sheet. Thus the edge of the patch to which the tab 19 of backing sheet material is adhesively secured can be lifted, with the tab 19 in situ, to facilitate gripping and handling of the patch as the patch is peeled from the backing sheet.

It will be recognised that the patch peeled from its backing sheet still has an area of the adhesive surface of the layer 10 covered by the tab 19, with the remainder of the adhesive surface exposed for placing against the skin of the user. The patch will be handled by its end carrying the tab 19 so that the adhesive surface of the layer 10 is not damaged by handling. After the patch has been placed in contact with the skin and smoothed to ensure that the exposed adhesive surface of the layer 10 attaches to the skin, the region of the patch still carrying the tab 19 can be lifted to peel the tab 19 from the layer 10 thus allowing the remainder of the layer 10 to be adhesively secured to the skin.

The tab 19 can be formed as part of the remainder of the backing sheet, but severed therefrom during the manufacturing process. Alternatively the tab 19 can be provided as an additional component in facial contact with the backing sheet 12 in use.

## Claims

1. A patch for transdermal therapy, the patch comprising at least one layer of sheet material (10) for adhesive attachment to the skin of a user, particulate material (11; 11a; 16) including particulate copper or an alloy thereof provided on the surface of said layer of sheet material presented to the skin of the user in use, and, at least one magnet (13; 13a) **characterised in that** said magnet (13; 13a) is disposed on the opposite surface of said layer of sheet material to said surface upon which said particulate material (11 11a; 16) is provided.

2. A patch as claimed in claim 1 **characterised in that** said magnet (13; 13a) is sandwiched between said layer (10) of sheet material and a second layer (14) of sheet material adhesively secured to said opposite surface of the first mentioned layer (10).

3. A patch as claimed in claim 1 or claim 2 **characterised in that** said particulate material also includes particulate zinc.

4. A patch as claimed in claim 1 or claim 2 **characterised in that** said patch includes a region of particulate zinc discrete from said particulate copper.

5. A patch as claimed in claim 4 **characterised in that** said discrete regions of copper and zinc communicate so as to be contactable by a common body of liquid electrolyte.

6. A patch as claimed in any one of claims 3 to 5 **characterised in that** said particulate zinc is disposed in a generally circular region (16) on said surface of said sheet (10) and is encircled by a generally circular annular region (11a) of particulate copper or an alloy thereof.

7. A patch as claimed in any one of claims 2 to 6 **characterised in that** a reinforcing strip (15) of sheet material overlies said magnet (13) in association with said second layer (14).

8. A patch as claimed in any one of claims 3 to 5 **characterised in that**, said magnet (13a) is annular and particulate zinc material (16) is disposed within the central aperture of the annular magnet.

9. A patch as claimed in claim 8 **characterised in that** said first layer (10) of sheet material is perforated (17) in alignment with the central aperture of the magnet (13a) to provide communication through said first layer (10) of sheet material between the particulate zinc (16) in the central aperture of the magnet and the particulate copper containing material (11) on the face of said layer (10) presented, in use, to the skin of the user.

10. A patch as claimed in any one of the preceding claims **characterised by** a peelable carrier sheet (12) upon which the patch is mounted by way of the adhesive surface of said layer (10) of sheet material, and a separate tab (19) of carrier sheet material peelably adhesively secured to a peripheral region of said first layer (10) of sheet material whereby said tab (19) can remain in place on said layer (10) of sheet material when the patch is peeled from the carrier sheet (12) to facilitate handling of the patch prior to said tab (19) being peeled from said layer (10) when the layer is applied to the skin of the user.

## Patentansprüche

1. Pflaster zur Transdermaltherapie, wobei das Pflaster aufweist: mindestens eine Lage des Schichtmaterials (10) für eine adhäsive Befestigung auf der Haut eines Benutzers; Teilchenmaterial (11, 11a; 16), das teilchenförmiges Kupfer oder eine Legierung davon umfaßt, das auf der Oberfläche der Lage des Schichtmaterials vorhanden ist, die zur Haut des Benutzers bei der Anwendung hin liegt; und mindestens einen Magneten (13; 13a), **dadurch gekennzeichnet, daß** der Magnet (13; 13a) auf der entgegengesetzten Oberfläche der Lage des Schichtmaterials zu der Oberfläche angeordnet ist, auf der das Teilchenmaterial (11; 11a; 16) bereitgestellt wird.

2. Pflaster nach Anspruch 1, **dadurch gekennzeichnet, daß** der Magnet (13; 13a) schichtartig zwischen der Lage (10) des Schichtmaterials und einer zweiten Lage (14) des Schichtmaterials angeordnet, ist, die auf der entgegengesetzten Oberfläche der ersten erwähnten Lage (10) adhäsiv gesichert ist.

3. Pflaster nach Anspruch 1 oder Anspruch 2, **dadurch gekennzeichnet, daß** das Teilchenmaterial ebenfalls teilchenförmiges Zink umfaßt.

4. Pflaster nach Anspruch 1 oder Anspruch 2, **dadurch gekennzeichnet, daß** das Pflaster einen Bereich aus teilchenförmigem Zink getrennt vom teilchenförmigen Kupfer umfaßt.

5. Plaster nach Anspruch 4, **dadurch gekennzeichnet, daß** die getrennten Bereiche von Kupfer und Zink in Verbindung stehen, um so durch einen gemeinsamen Körper des flüssigen Elektrolyts in Kontakt kommen zu können.

6. Pflaster nach einem der Ansprüche 3 bis 5, **dadurch gekennzeichnet, daß** das teilchenförmige Zink in einem im allgemeinen kreisförmigen Bereich (16) auf der Oberfläche der Schicht (10) angeordnet und durch einen im allgemeinen kreisförmigen, ringförmigen Bereich (11a) des teilchenförmigen Kupfers oder einer Legierung davon umschlossen wird.

7. Pflaster nach einem der Ansprüche 2 bis 6, **dadurch gekennzeichnet, daß** ein Verstärkungsstreifen (15) des Schichtmaterials über dem Magneten (13) in Verbindung mit der zweiten Lage (14) liegt.

8. Pflaster nach einem der Ansprüche 3 bis 5, **dadurch gekennzeichnet, daß** der Magnet (13a) ringförmig ist und das teilchenförmige Zinkmaterial (16) innerhalb der mittleren Öffnung des ringförmigen Magneten angeordnet ist.

9. Pflaster nach Anspruch 8, **dadurch gekennzeichnet, daß** die erste Lage (10) des Schichtmaterials in Ausrichtung mit der mittleren Öffnung des Magneten (13a) perforiert (17) ist, um eine Verbindung durch die erste Lage (10) des Schichtmaterials zwischen dem teilchenförmigen Zink (16) in der mittleren Öffnung des Magneten und dem das teilchenförmige Kupfer enthaltenden Material (11) auf der Seite der Lage (10) zu liefern, die bei Anwendung zur Haut des Benutzers hin liegt.

10. Pflaster nach einem der vorhergehenden Ansprüche, **gekennzeichnet durch** eine abziehbare Trägerschicht (12), auf der das Pflaster mittels der adhäsiven Oberfläche der Lage (10) des Schichtmaterials befestigt wird, und eine separate Lasche (19) des Trägerschichtmaterials, die abziehbar adhäsiv an einem Umfangsbereich der ersten Lage (10) des Schichtmaterials gesichert ist, wobei die Lasche (19) an Ort und Stelle auf der Lage (10) des Schichtmaterials verbleiben kann, wenn das Pflaster von der Trägerschicht (12) abgezogen wird, um die Handhabung des Pflasters vor dem Abziehen der Lasche (19) von der Lage (10) zu erleichtern, wenn die Lage auf die Haut des Benutzers aufgebracht wird.

## Revendications

1. Timbre pour thérapie transdermique, le timbre comprenant au moins une couche de matériau en feuille (10) en vue d'une fixation par adhésion sur la peau d'un utilisateur, un matériau particulaire (11; 11a; 16) englobant du cuivre particulaire ou un alliage correspondant appliqué sur la surface de ladite couche de matériau en feuille présentée en service à la peau de l'utilisateur, et au moins un aimant (13; 13a), **caractérisé en ce que** ledit aimant (13; 13a) est agencé sur la surface de ladite couche de matériau en feuille opposée à ladite surface sur laquelle est appliqué ledit matériau particulaire (11; 11a; 16).

2. Timbre selon la revendication 1, **caractérisé en ce que** ledit aimant (13; 13a) est agencé en sandwich entre ladite couche (10) de matériau en feuille et une deuxième couche (14) de matériau en feuille fixée par adhésion sur ladite surface opposée de ladite première couche (10).

3. Timbre selon les revendications 1 ou 2, **caractérisé en ce que** ledit matériau particulaire englobe aussi du zinc particulaire.

4. Timbre selon les revendications 1 ou 2, **caractérisé en ce que** ledit timbre englobe une région de zinc particulaire distincte dudit cuivre particulaire.

5. Timbre selon la revendication 4, **caractérisé en ce que** lesdites régions distinctes de cuivre et de zinc sont en communication, de sorte à pouvoir être contactées par un corps commun d'électrolyte liquide.

6. Timbre selon l'une quelconque des revendications 3 à 5, **caractérisé en ce que** ledit zinc particulaire est agencé dans une région généralement circulaire (16) sur ladite surface de ladite feuille (10) et est encerclé par une région annulaire généralement circulaire (11a) de cuivre particulaire ou d'un alliage correspondant.

7. Timbre selon l'une quelconque des revendications 2 à 6, **caractérisé en ce qu'**une bande de renforcement (15) de matériau en feuille est superposée audit aimant (13), en association avec ladite deuxième couche (14).

8. Timbre selon l'une quelconque des revendications 3 à 5, **caractérisé en ce que** ledit aimant (13a) est annulaire, le matériau de zinc particulaire (16) étant agencé dans l'ouverture centrale de l'aimant annulaire.

9. Timbre selon la revendication 8, **caractérisé en ce que** ladite première couche (10) de matériau en feuille comporte des perforations (17) alignées avec l'ouverture centrale de l'aimant (13a) pour établir une communication à travers ladite première couche (10) de matériau en feuille entre le zinc particulaire (16) dans l'ouverture centrale de l'aimant et le matériau contenant le cuivre particulaire (11) sur la face de ladite couche (10) présentée en service à la peau de l'utilisateur.

10. Timbre selon l'une quelconque des revendications précédentes, **caractérisé par** une feuille de support pelable (12) sur laquelle le timbre est monté par l'intermédiaire de la surface adhésive de ladite couche (10) de matériau en feuille, et une patte séparée (19) de matériau en feuille de support fixée par adhésion, et de manière à pouvoir être détachée par pelage, sur une région périphérique de ladite première couche (10) de matériau en feuille, ladite patte (19) pouvant ainsi rester dans sa position sur ladite couche (10) de matériau en feuille lorsque le timbre est détaché par pelage de la feuille de support (12) pour faciliter la manipulation du timbre avant le détachement par pelage de ladite patte (19) de ladite couche (10) lors de l'application de la couche sur la peau de l'utilisateur.
